# EUROPÄISCHE PATENTSCHRIFT

(11) **EP 2 192 956 B1**
(45) Veröffentlichungstag und Bekanntmachung des Hinweises auf die Patenterteilung: **07.06.2017**
(21) Anmeldenummer: 08831932.2
(22) Anmeldetag: 19.09.2008
(51) Int. Cl.: A61Q 19/00, A61K 36/87, A61K 8/97, A61K 36/185, A61K 36/28, A61K 36/48, A61K 36/71

(54) **KOSMETISCHE ODER DERMATOLOGISCHE ZUSAMMENSETZUNG ZUR TOPISCHEN VERWENDUNG**
COSMETIC OR DERMATOLOGICAL COMPOSITION FOR TOPICAL APPLICATION
COMPOSITION COSMÉTIQUE OU DERMATOLOGIQUE POUR UTILISATION TOPIQUE

(30) Priorität: 19.09.2007 DE 102007044916
(43) Veröffentlichungstag der Anmeldung: 09.06.2010
(73) Patentinhaber: Bionorica SE, 92318 Neumarkt (DE)
(72) Erfinder: WUTTKE, Wolfgang, 37120 Bovenden (DE); SEIDLOVA-WUTTKE, Dana, 31720 Bovenden (DE)
(74) Vertreter: Simandi, Claus
(86) Internationale Anmeldenummer: PCT/DE2008/001561
(87) Internationale Veröffentlichungsnummer: WO 2009/036751

(56) Entgegenhaltungen:
- EP-A- 1 992 322
- US-A1- 2003 007 939
- US-A1- 2004 223 932
- DATABASE WPI Week 200308 Thomson Scientific, London, GB; AN 2003-076347 XP002517377 & CN 1 160 537 A (YUWEN J) 1. Oktober 1997 (1997-10-01)
- DATABASE WPI Week 200210 Thomson Scientific, London, GB; AN 2002-067295 XP002517378 & CN 1 174 716 A (ZHANG Z) 4. März 1998 (1998-03-04)
- DATABASE WPI Week 200207 Thomson Scientific, London, GB; AN 2002-049895 XP002517379 & CN 1 310 996 A (CUI W) 5. September 2001 (2001-09-05)
- DATABASE WPI Week 200719 Thomson Scientific, London, GB; AN 2007-185647 XP002517380 & CN 1 843 419 A (CHINESE PLA GEN HOSPITAL) 11. Oktober 2006 (2006-10-11)
- DATABASE WPI Week 200658 Thomson Scientific, London, GB; AN 2006-565955 XP002517381 & KR 2005 052 839 A (AMOREPACIFIC CORP) 7. Juni 2005 (2005-06-07)
- DATABASE WPI Week 199622 Thomson Scientific, London, GB; AN 1996-217148 XP002517382 -& JP 08 081336 A (POLA CHEM IND INC) 26. März 1996 (1996-03-26)
- DATABASE WPI Week 200709 Thomson Scientific, London, GB; AN 2007-086834 XP002517383 -& JP 2006 342068 A (OGAWA KORYO KK) 21. Dezember 2006 (2006-12-21)
- DATABASE WPI Week 200348 Thomson Scientific, London, GB; AN 2003-511210 XP002517384 & KR 2003 021 991 A (LEE D J) 15. März 2003 (2003-03-15)
- DATABASE WPI Week 199745 Thomson Scientific, London, GB; AN 1997-481108 XP002517385 & CN 1 120 952 A (CHEN X) 24. April 1996 (1996-04-24) in der Anmeldung erwähnt
- DATABASE WPI Week 200776 Thomson Scientific, London, GB; AN 2007-812176 XP002517386 & KR 2007 000 675 A (LG HOUSEHOLD & HEALTHCARE LTD) 3. Januar 2007 (2007-01-03)
- DATABASE WPI Week 200772 Thomson Scientific, London, GB; AN 2007-768068 XP002517405 -& JP 2007 238509 A (ASAHI BREWERIES LTD) 20. September 2007 (2007-09-20)
- WUTTKE, W ET AL.: "Dermal application of a Cimicifuga racemosa (CR)- containing cream has beneficial effects on acne" EUROPEAN JOURNAL OF INTEGRATIVE MEDICINE, [Online] Bd. 1, Nr. S1, 1. November 2008 (2008-11-01), Seite 40, XP002517374 Gefunden im Internet: URL:http://www.sciencedirect.com/science?_ ob=MImg&_imagekey=B984N-4TSW8DC-2F-1&_cdi= 59075&_user=987766&_orig=browse&_coverDate =11%2F30%2F2008&_sk=999989999.8998&view=c& wchp=dGLbVtz-zSkWA&md5=84b1e511a44b248ab15 d63e5f792ed86&ie=/sdarticle.pdf> [gefunden am 2009-02-26]
- ANONYMOUS: "Phytessence Cimicifuga" MATERIAL DATA SHEET ART. NR. - NA2186C, [Online] 2009, XP002517375 Gefunden im Internet: URL:http://www.in-cosmetics.com/ExhibitorL ibrary/744/Phytessence_Cimicifuga_NA21860_ MDS_page_1_3.pdf> [gefunden am 2009-03-02]
- SEIDLOVA-WUTTKE ET AL: "Inhibition of 5alpha-reductase in the rat prostate by Cimicifuga racemosa" 23. November 2006 (2006-11-23), MATURITAS, ELSEVIER SCIENCE PUBLISHERS IRELAND LTD, IR, PAGE(S) S75 - S82 , XP005753627 ISSN: 0378-5122 das ganze Dokument

## Beschreibung

Die Erfindung betrifft eine kosmetische oder dermatologische Zusammensetzung, vorzugsweise auf reiner Pflanzenbasis, zur topischen Verwendung enthaltend als primären natürlichen Wirkstoff Cimicifuga sowie deren Verwendung zur Prophylaxe und Behandlung von Hauterkrankungen, insbesondere Akne, Seborrhoe, atopisches Ekzem (Neurodermitis), Hirsutismus, Schuppenflechte (Psoriasis) sowie trockener / atopischer Haut des Menschen.

An einer modernen kosmetischen oder dermatologischen Zusammensetzung, vorzugsweise auf reiner Pflanzenbasis, zur topischen Verwendung sind hohe Anforderungen zu stellen.

Die Haut übt als das größte Organ des Menschen zahlreiche lebenswichtige Funktionen aus. Sie schützt z.B. vor Kälte, Hitze, Strahlung, dem Einwirken chemischer Substanzen und Krankheitserregern. Sofern die Haut diese Barrierefunktion nicht mehr ausreichend erfüllt, können lokale Irritationen oder auch ganzkörperliche Beschwerden erfolgen.

Die Haut, insbesondere die Epidermis, ist als Barriereorgan des menschlichen Organismus in besonderem Maße äußeren Einwirkungen unterworfen. Diese Barrierefunktion wird unter anderem von Hautlipiden aufrechterhalten. Diese epidermalen Lipide wie z.B. Glycosphingolipide, Ceramide, Sterine und Sterinester, Fettsäuren, Triglyceride, n-Alkane oder verschiedenen polaren Lipiden werden im Keratinisierungsprozess freigesetzt.

In einem optimalen Zustand der Haut liegt ein ausgewogenes Verhältnis von Hautlipiden und Hautfeuchtigkeit vor. Durch dieses Gleichgewicht werden wichtige Eigenschaften der Haut, wie das Penetrationsvermögen, Wasserbindungsvermögen, Elastizität, Regenerationsfähigkeit oder die Widerstandsfähigkeit gegen Umwelteinflüsse und Noxen unterschiedlichster Art, bestimmt. Den Hautlipiden, besonders den Oberflächenlipiden, ist dabei eine übergeordnete Bedeutung zuzuschreiben.

Der äußere Lipidfilm der Haut stellt eine sehr komplex zusammengesetzte Emulsion aus verschiedenen Lipiden und dem Sekret der Schweißdrüsen, wie Harnstoff, Fettsäuren, anorganische Salze und Wasser, dar. Die Bestandteile der Ölphase stammen vorrangig aus den Absonderungen der Talgdrüsen, die u.a. Squalen, Cholesterol und Cholesterolester, Ester vom Wachstyp, Triglyceride und freie Fettsäuren enthalten.

Im Stand der Technik ist in US 2003007939 das "black cohosh" als geringer Nebenbestandteil in einer pharmazeutischen Zusammensetzung zur Behandlung von Hautkrankheiten offenbart. CN 1196037 offenbart die orale Applikation von Cimicifuga zur Behandlung von Akne in Tablettenform. Nachteilig ist jedoch die geringe Wirksamkeit im Wege der oralen Applikation. DE 3641220 offenbart den Einsatz von Cimicifuga neben anderen Bestandteilen zur Behandlung von Hautkrankheiten, wobei jedoch Cimicifuga lediglich in homöopathischen Dosen verabreicht wird.

In CN 1120952 A als auch CN 1310996 A werden Mehrkomponentenmischungen, die u.a. Cimicifuga enthalten, zur Behandlung der Akne offenbart. Cimicifuga ist jedoch nicht der primäre Wirkstoff, sondern lediglich ein Nebenbestandteil. Zudem ist in CN 1120952 A der Hauptbestandteil dieser Mischung in einer Vaseline-Creme mit nachteiligen Eigenschaften für die Haut enthalten. Vaseline ist zwar ein kostengünstiges Grundmaterial; es führt jedoch zu toxischen Wirkungen in der Haut.

CN 1843419 A offenbart Zusammensetzungen aus 5 bis 20 Teilen Rhabarber und 20 bis 40 Teilen Cimicifuga racemosa, insbesondere zur Behandlung von Herpes-Virusinfektionen.

Die Anmelderin konnte bereits nachweisen, dass Cimicifuga die 5-alpha-Reduktase inhibieren kann (EP1064009B1, EP1427430B1). Daher sind Cimicifuga-Extrakte ebenfalls zur Therapie des Prostatakarzinoms geeignet. 5-alpha-Reduktase ist ein Enzym, welches im Serum zirkulierendes Testosteron lokal in Prostata und in den Hautanhangsorganen zu 5-alpha-Dehydrotestosteron (5-alpha-DHT) reduziert. Dieses ist das eigentlich wirksame Androgen. In der Haut (exklusive Kopfhaut) stimuliert jedoch 5-alpha-DHT die Talgproduktion und das Haarwachstum.

Der vorliegenden Erfindung liegt die Aufgabe zugrunde, eine kosmetische oder dermatologische Zusammensetzung zur topischen Verwendung bereitzustellen, die neben der Pflege der Haut geeignet ist, Hauterkrankungen, insbesondere Akne, Psoriasis, Seborrhoe, atopisches Ekzem (Neurodermitis), Schuppenflechte sowie trockener / atopischer Haut des Menschen zu behandeln und diesbezüglich eine verbesserte kosmetische oder dermatologische Zusammensetzung in der topischen Verwendung zu erzielen.

Die Aufgabe wird durch eine dermatologische Zubereitung zur topischen Verwendung gemäß Anspruch 1 gelöst, die 1,0 - 50 Gew.-% als primären Wirkstoff Cimicifuga enthält. In weiteren Ausführungsformen werden vorzugsweise 2,0 - 30 Gew.-%, besonders bevorzugt 2,5 - 20 Gew.-% Cimicifuga sowie besonders bevorzugt 2,5 - 10 Gew.-% in der erfindungsgemäßen Zubereitung eingesetzt. Diese Ausführungsformen können zur üblichen Pflege der Haut als auch gleichzeitig zur Behandlung von Hauterkrankungen und trockener / atopischer Haut effizient und komplex verwendet werden und beste Ergebnisse erzielen.

Die ausgewählten Bereiche des erfindungsgemäßen primären Wirkstoff Cimicifuga erlauben besonders vorteilhaft insbesondere im Rahmen der topischen Verwendung eine optimale Wirkung auf die 5-alpha-Reduktase. Vorzugsweise in der Form einer Hautcreme oder Emulsion, Salbe (Paste, Creme, Gel) oder Lotion kann die erfindungsgemäße Zusammensetzung auf die Haut aufgetragen werden.

Im Rahmen dieser Erfindung ist "Cimicifuga", nämlich Cimicifuga racemosa (Traubensilberkerze aus der Familie der Hahnenfußgewächse (Ranunculaceae))und zwar ein Extrakt oder Trockenextrakt aus dem Wurzelstock ("Rhizoma cimicifugae racemosae", siehe EP1427430B1).

"Primärer Wirkstoff" bedeutet, dass der Wirkstoff Cimicifuga neben anderen Wirkstoffen mengenmäßig vorrangig in der erfindungsgemäßen Zusammensetzung vorliegt.

Dementsprechend können sekundäre Wirkstoffe in einer Wirkstoffkombination neben dem primären Wirkstoff Cimicifuga vorliegen. Solche Wirkstoffe sind vorzugsweise Extrakte aus Soja, Rotklee, Hopfen (Humulus, insbesondere Humulus lupulus), Weinschalen und Calendula, insbesondere solche Extrakte, die einen hohen Anteil an Isoflavonen enthalten.

Diese sekundären Wirkstoffe wirken nach perkutaner Applikation fördernd auf die Wirkung des Cimicifuga.

In einer besonders bevorzugten Ausführungsform basiert die erfindungsgemäße Zusammensetzung auf reiner Pflanzenbasis.

Eine solche Ausführungsform kann z.B. vorteilhaft unverseifbare Bestandteile aus mindestens einem weiteren pflanzlichen Öl enthalten und insbesondere keine Mineralöle oder Fette aus Rückständen der Erdöldestillation (z.B. wie Vaseline u.v.a.).

Im Rahmen dieser Erfindung wird unter "unverseifbare Bestandteile aus mindestens einem weiteren pflanzlichen Öl" die Gesamtheit der fettlöslichen und wasserunlöslichen organischen Bestandteile aus mindestens einer Pflanze verstanden, die durch eine Verseifungsreaktion nicht in Salze überführt werden können (z.B. nach DIN 53 900, technische Vorschrift, Germany). Pflanzliche Fette enthalten zumeist 0,1 - 5 Gew.-% unverseifbare Bestandteile. Die Zusammensetzung dieser unverseifbaren Bestandteile können nicht abschließend sein: aromatische Verbindungen, Terpene, Tocopherole, Carotine, C-16- bis C-22-Kohlenwasserstoffe, Terpenalkohole, C-16 bis C-22-Alkohole sowie verschiedene Sterine, z.B. Cholesterin, alpha-Sitosterin, Stigmasterin, Campesterin, Brassicasterin und Phytosterine. Diese Bestandteile sind nicht als Wirkstoffe zu verstehen, sondern unterstützen die topische Verwendung der erfindungsgemäßen Zusammensetzung.

Im Rahmen dieser Erfindung wird unter dem Begriff "atopisches Ekzem" (auch: atopische Dermatitis bzw. "Neurodermitis") eine immer häufiger auftretende schubweise Hauterkrankung, die insbesondere Heranwachsende betrifft verstanden. Die Haut eines an Neurodermitis Erkrankten ist in der Akutphase hoch sensibel und kann allergisch auf psychische und physische Stressoren sowie eine ganze Reihe von individuell unterschiedlichen Umweltfaktoren und -noxen reagieren. In der Akutphase ist die Haut entzündet und der Betroffene leidet ganz besonders unter dem quälenden Juckreiz. Soweit bekannt, liegen der Neurodermitis keine Erkrankung der inneren Organe zugrunde, sondern entzündliche freie radikale produzierende Prozesse der äußeren Hautschichten, so dass durch externe Applikation von Salben, Cremes oder Gelen hier Einfluss positiv genommen werden kann.

In einer weiteren Ausführungsform kann die erfindungsgemäße kosmetische oder dermatologische Zubereitung zur topischen Verwendung in Form einer Salbe, Creme, Gel, Lotion (Hautmilch), Paste oder vorzugsweise Emulsion erfolgen. Ebenfalls sind wasserfreie Systeme möglich.

Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert. Sind die beiden Flüssigkeiten Wasser und Öl und liegen Öltröpfchen fein verteilt in Wasser vor, so handelt es sich um eine Öl-in-Wasser-Emulsion (O/W-Emulsion). Der Grundcharakter einer O/W-Emulsion ist durch das Wasser geprägt. Bei einer Wasser-in-Öl-Emulsion (W/O-Emulsion) handelt es sich um das umgekehrte Prinzip, in dem der Grundcharakter hier durch das Öl bestimmt wird. Weiterhin sind Mischsysteme wie Wasser-in-Öl-in-Wasser-Emulsionen (W/O/W-Emulsion) und Öl-in-Wasser-in-Öl-Emulsionen (O/W/O-Emulsionen) bekannt. Alle genannten Emulsionen sind erfindungsgemäß geeignet.

Zu den erfindungsgemäß geeigneten wasserfreien Systemen gehören reine Ölzubereitungen wie z.B. Hautöle. Ebenfalls einsetzbare Pasten enthaltend die erfindungsgemäße Zubereitung, zeichnen sich dadurch aus, dass sie aus den gleichen oder ähnlichen Bestandteilen wie eine Emulsion besteht, aber im Wesentlichen wasserfrei sind. Im Rahmen der vorliegenden Erfindung werden die Begriffe Ölphase und Lipidphase synonym verwendet. In einer weiteren bevorzugten Ausführungsform kann die erfindungsgemäße Zubereitung als weiteren Bestandteil einen Emulgator beinhalten. In einer ganz bevorzugten Ausführung kann dieser Emulgator ein O/W-Emulgator sein.

Emulgatoren können vorteilhaft gewählt werden aus der Gruppe der nichtionischen, anionischen, kationischen oder amphoteren Emulgatoren.

Als nichtionischer Emulgator können verschiedene Emulgatoren aus den Gruppen der Partialfettsäureester, Fettalkohole, Sterole, Polyethylengylcole wie z.B. ethoxylierte Fettsäuren, ethoxylierte Fettalkohole und ethoxylierte Sorbitanester, Zuckeremulgatoren, Polyglycerinemulgatoren oder Silikonemulgatoren Verwendung finden.

Als anionischer Emulgator können verschiedene Emulgatoren aus den Gruppen der Seifen z. B. Natriumstearat, Fettalkoholsulfate, Mono-, Di- und Trialkylphosphosäureester und deren Ethoxylate, Fettsäure Lactat Ester, Fettsäure Citrat Ester oder Fettsäure Citroglycerinester Verwendung finden.

Als kationischer Emulgator können beispielsweise quaternäre Ammoniumverbindungen mit einem langkettigen aliphatischen Rest z. B. Distearyldimonium Chloride eingesetzt werden.

Unter den amphoteren Emulgatoren können verschiedene Emulgatoren aus den Gruppen Alkylamininoalkancarbonsäuren, Betaine, Sulfobetaine oder Imidazolinderivate Verwendung finden.

Erfindungsgemäß bevorzugt sind natürlich vorkommende Emulgatoren, zu denen beispielsweise Bienenwachs, Wollwachs, Lecithin und Sterole u.a. gehören, die ebenfalls bei der Herstellung einer erfindungsgemäßen Zubereitung eingesetzt werden können. In einer bevorzugten Formulierung der erfindungsgemäßen Zubereitung können O/W-Emulgatoren ausgewählt werden aus der Gruppe der Pflanzenproteinhydrolysaten und deren Derivate.

Im Sinne der vorliegenden Erfindung können ferner vorteilhaft Substanzen als Zusatzstoffe enthalten sein, ausgewählt aus der Gruppe der Ester aus gesättigten und/oder ungesättigten, verzweigten und/oder unverzweigten Alkancarbonsäuren und/oder Alkencarbonsäuren mit einer Kettenlänge von 3 - 30 Kohlenstoffatomen und gesättigten und/oder ungesättigten, verzweigten und/oder unverzweigten Alkoholen einer Kettenlänge von 3 - 30 Kohlenstoffatomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esterole können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononyliso-nonanoat, 2-Ethylhexylpalmitat, 2-Ethylhexyllaurat, 2- Hexyldecylstearat, 2-Octyldodecyl-palmitat, Oleyloleat, Oleylerucat, Erucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 - 24 C-Atomen, insbesondere 12 - 18 C-Atomen. Die Fettsäuretriglyceride können beispielsweise vorteilhaft gewählt werden aus der Gruppe der synthetischen, halbsynthetischen und natürlichen Öle.

Daher betrifft die Erfindung ebenfalls eine erfindungsgemäße Zubereitung, die als Zusatzstoffe, beispielsweise Jojobaöl, Olivenöl, Sonnenblumenöl, Sojaöl, Erdnussöl, Rapsöl, Mandelöl, Palmöl, Kokosöl, Palmkernöl oder dergleichen enthält. Ganz besonders bevorzugt sind pflanzliche Öle, die mindestens 6 Gew. -% an mehrfach ungesättigten Fettsäuren aufweisen.

In einer weiteren bevorzugten Ausführungsform enthalten die erfindungsgemäßen Zubereitungen mindestens 1 Gew. -%, vorzugsweise 1 - 30 Gew. -%, besonders bevorzugt 2 - 15 Gew. - % oder gar 5 - 10 Gew. -% eines solchen, mindestens eines solchen pflanzlichen Öls als Zusatzstoff.

Insbesondere können in den erfindungsgemäßen Zubereitungen zusätzlich als Hilfs- oder Zusatzstoff Antioxidantien und/oder Radikalfänger zugesetzt werden. Vorteilhaft werden solche Antioxidantien gewählt aus der Gruppe der lipophilen Systeme, beispielsweise: natürliche und synthetische Tocopherole, Nordihydroguajaretsäure, Coniferylbenzoat, Butylhydroxyanisol, Butlyhydroxytoluol, Gallussäureester und verschiedenen antioxidative Pflanzen-Extrakte. Unter den hydrophilen Systemen sind besonders vorteilhaft anorganische Schwefelverbindungen, Natriumhydrogensulfit, Cystein oder Ascorbinsäure zu verwenden.

Die erfindungsgemäßen kosmetischen und dermatologischen Zubereitungen können weiterhin kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, weichmachende, anfeuchtende und/oder feuchthaltende Substanzen oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

In einer weiteren besonderen Ausführungsform ist die erfindungsgemäße Zubereitung im Wesentlichen aus natürlich vorkommenden Inhaltsstoffen zusammengesetzt, wie oben genannt.

Des Weiteren betrifft die Erfindung die Verwendung der erfindungsgemäßen Zusammensetzung zur Prophylaxe und Behandlung von Hauterkrankungen, insbesondere Akne, Seborrhoe, atopisches Ekzem (Neurodermitis), Hirsutismus, Schuppenflechte (Psoriasis) sowie trockener /atopischer Haut des Menschen.

Zudem betrifft die Erfindung ein Arzneimittel zur Behandlung von Hauterkrankungen am Menschen, insbesondere zur Prophylaxe und Behandlung von Hauterkrankungen, insbesondere Akne, Seborrhoe, atopisches Ekzem (Neurodermitis), Hirsutismus, Schuppenflechte (Psoriasis) sowie trockener /atopischer Haut des Menschen. Das Arzneimittel kann zu diesen Zwecken äußerlich auf die Haut des Menschen aufgetragen werden.

Zur Herstellung der erfindungsgemäßen Extrakte und deren Kombinationen (primärer Wirkstoff und sekundärer Wirkstoff) aus den Pflanzen wird auf die technischen Lehren, die Gegenstand von EP 1368605B1, EP 0753306B1 sind, verwiesen.

Nachfolgend einige Ausführungsbeispiele, ohne die Erfindung auf diese Beispiele zu beschränken.

**O/W-Emulsion mit Cimicifuga-Extrakt:**

| | |
|---|---|
| Glycerinmonostearat selbstemulg. | 3,0% |
| Sorbitanstearat | 2,0% |
| Cetylalkohol | 3,0% |
| Lanolinalkohol | 5,0% |
| Isopropylstearat | 3,0% |
| Pflanzenöle | 7,0% |
| Silikonöle | 5,0% |
| Cimicifuga Extrakt | 3,0% |
| Glyzerin | 5,0% |
| Calendula Extrakt | 2,5% |
| Polyacrylsäure | 0,2% |
| Triethanolamin | 0,3% |
| Konservierungsmittel | 0,4% |
| Parfum | 0,3% |
| Wasser ad | 100,0% |

**W/O-Emulsion mit Cimicifuga-Extrakt:**

| | |
|---|---|
| Glyceryl Sorbitan Isostearat | 8,0% |
| Methyl Glucose Dioleat | 2,0% |
| Talgglyceride | 2,0% |
| Lanolinalkohol | 2,0% |
| Capryl-/Capringlyceride | 5,0% |
| Isopropylstearat | 8,0% |
| Silkonöl | 2,0% |
| Pflanzenöle | 5,0% |
| Cimicifuga Extrakt | 4,0% |
| Calendula Extrakt | 3,0% |
| Glyzerin | 4,0% |
| Konservierungsmittel | 0,4% |
| Parfum | 0,4% |
| Wasser ad | 100,0% |

**Gesichtspflege Gel mit Cimicifuga:**

| | |
|---|---|
| Carbopol | 0,8% |
| Cellulose Gum | 0,1% |
| Butylenglykol | 6,0% |
| Natrium Hydroxid | 0,3% |
| Cimicifuga Extrakt | 3,5% |
| Calendula Extrakt | 2,5% |
| Konservierungsmittel | 0,4% |
| Ethylalkohol | 8,0% |
| Parfum | 0,2% |
| Wasser ad | 100,0% |

**Gesichtslotion mit Cimicifuga:**

| | |
|---|---|
| Butylenglykol | 6,0% |
| Emulgator | 2,0% |
| Ethylalkohol | 25,0% |
| Cimicifuga Extrakt | 4,0% |
| Calendula Extrakt | 3,0% |
| Parfum | 0,3% |
| Wasser ad | 100,0% |

In allen Beispielen ist Cimicifuga der primäre Wirkstoff und kann von einem sekundären Wirkstoff, wie Soja, Rotklee, Hopfen, Weinschalen an Stelle von oder Teil von Calendula in der Wirkung unterstützt werden.

## Patentansprüche

1. Dermatologische Zusammensetzung enthaltend den primären Wirkstoff 1,0 - 50 Gew.-% eines Cimicifuga racemosa-Extraktes aus dem Wurzelstock zur topischen Verwendung in der Prophylaxe und Behandlung von Hauterkrankungen ausgewählt aus der Gruppe Akne, Seborrhoe, atopisches Ekzem (Neurodermitis), Hirsutismus, Schuppenflechte (Psoriasis) sowie trockener /atopischer Haut des Menschen und weitere Hilfs- und/oder Zusatzstoffe.

2. Dermatologische Zusammensetzung zur topischen Verwendung nach Anspruch 1, **dadurch gekennzeichnet, dass** ein Cimicifuga racemosa-Extrakt aus dem Wurzelstock mit 2,0 - 30 Gew.-%, 2,5 - 20 Gew.-% oder 2,5 - 10 Gew.-% enthalten ist.

3. KDermatologische Zusammensetzung zur topischen Verwendung nach einem der Ansprüche 1 oder 2, enthaltend zusätzlich Extrakte aus Soja, Rotklee, Hopfen, Weinschalen und Calendula.

4. Dermatologische Zusammensetzung zur topischen Verwendung nach einem der Ansprüche 1 bis 3, **dadurch gekennzeichnet, dass** 0,1 - 15 Gew.-% unverseifbare Bestandteile aus mindestens einem weiteren pflanzlichen Öl enthalten ist.

5. Dermatologische Zusammensetzung zur topischen Verwendung nach einem der Ansprüche 1 bis 4, in Form einer Creme, Gel, Lotion, Paste, Salbe, Hautöl oder Emulsion.

6. Dermatologische Zusammensetzung zur topischen Verwendung nach einem der Ansprüche 1 bis 5, **dadurch gekennzeichnet, dass** als Zusatzstoff mindestens ein weiteres pflanzliches Öl enthalten ist, das mindestens 6 Gew. -% an mehrfach ungesättigten Fettsäuren enthält.

7. Dermatologische Zusammensetzung zur topischen Verwendung nach einem der Ansprüche 1 bis 6, **dadurch gekennzeichnet, dass** ein oder mehrere Zusatzstoffe ausgewählt sind aus der Gruppe Jojobaöl, Olivenöl, Sonnenblumenöl, Sojaöl, Erdnussöl, Rapsöl, Mandelöl, Palmöl, Kokosöl, Palmkernöl.

8. Dermatologische Zusammensetzung zur topischen Verwendung nach einem der Ansprüche 1 bis 7, **dadurch gekennzeichnet, dass** ein O/W-Emulgator, insbesondere ein Pflanzenproteinhydrolysat, enthalten ist.

9. Arzneimittel enthaltend eine dermatologische Zusammensetzung nach einem der Ansprüche 1 bis 8 zur topischen Verwendung in der Prophylaxe und Behandlung von Hauterkrankungen ausgewählt aus der Gruppe Akne, Seborrhoe, atopisches Ekzem (Neurodermitis), Hirsutismus, Schuppenflechte (Psoriasis) sowie trockener /atopischer Haut.

## Claims

1. A dermatological composition comprising the primary active ingredient 1.0 to 50% by weight of Cimicifuga racemosa-extract from the rootstock for topical use in the prophylaxis and treatment of skin diseases selected from the group consisting of acne, seborrhea, atopic eczema (neurodermatitis), hirsutism, psoriasis and dry/atopic skin of humans, and further auxiliary agents and/or additives.

2. The dermatological composition for topical use according to claim 1, **characterized in that** it comprises a Cimicifuga racemosa-extract from the rootstock with 2.0 to 30% by weight, 2.5 to 20% by weight, or 2.5 to 10% by weight.

3. The dermatological composition for topical use according to claim 1 or 2, additionally comprising extracts of soy, red clover, hops, grape skins and Calendula.

4. The dermatological composition for topical use to any one of claims 1 to 3, **characterized in that** it contains 0.1 to 15% by weight of unsaponifiable constituents of at least one further vegetable oil.

5. The dermatological composition for topical use according to any one of claims 1 to 4, in the form of a cream, gel, lotion, paste, ointment, skin oil, or emulsion.

6. The dermatological composition for topical use according to any one of claims 1 to 5, **characterized in that** it comprises at least one further vegetable oil, which includes at least 6% by weight of polyunsaturated fatty acids, as an additive.

7. The dermatological composition for topical use according to any one of claims 1 to 6, **characterized in that** it comprises one or more additives selected from the group consisting of jojoba oil, olive oil, sunflower oil, soy bean oil, peanut oil, rapeseed oil, almond oil, palm oil, coconut oil, or palm kernel oil.

8. The dermatological composition for topical use according to any one of claims 1 to 7, **characterized in that** it comprises an O/W emulsifier, particularly a plant protein hydrolysate.

9. A drug comprising a dermatological composition according to any one of claims 1 to 8 for topical use in the prophylaxis and treatment of skin diseases selected from the group consisting of acne, seborrhea, atopic eczema (neurodermatitis), hirsutism, psoriasis, and dry/atopic skin.

## Revendications

1. Composition dermatologique contenant la matière active primaire à raison de 1,0 à 50 % en poids d'un extrait de Cimicifuga racemosa provenant du rhizome pour une utilisation topique dans la prophylaxie et le traitement de maladies de la peau sélectionnées dans le groupe constitué par l'acné, la séborrhée, l'eczéma atopique (neurodermite), l'hirsutisme, le psoriasis (psoriasis), ainsi que la peau sèche /atopique de l'humain, et d'autres produits auxiliaires et/ou additifs.

2. Composition dermatologique pour l'utilisation topique selon la revendication 1, **caractérisée en ce qu'**un extrait de Cimicifuga racemosa provenant du rhizome est contenu à raison de 2,0 à 30 % en poids, de 2,5 à 20 % en poids ou de 2,5 à 10 % en poids.

3. Composition dermatologique pour l'utilisation topique selon l'une des revendications 1 ou 2, contenant en outre des extraits provenant de soja, de trèfle rouge, de houblon, de pellicules de raisin et de calendula.

4. Composition dermatologique pour l'utilisation topique selon l'une des revendications 1 à 3, **caractérisée en ce que** 0,1 à 15 % en poids de composants non saponifiables provenant d'au moins une huile végétale supplémentaire sont contenus.

5. Composition dermatologique pour l'utilisation topique selon l'une des revendications 1 à 4, sous la forme d'une crème, d'un gel, d'une lotion, d'une pâte, d'une pommade, d'une huile pour la peau ou d'une émulsion.

6. Composition dermatologique pour l'utilisation topique selon l'une des revendications 1 à 5, **caractérisée en ce qu'**en tant que produit auxiliaire au moins une huile végétale supplémentaire est contenue, qui contient au moins 6 % en poids d'acides gras plusieurs fois insaturés.

7. Composition dermatologique pour l'utilisation topique selon l'une des revendications 1 à 6, **caractérisée en ce qu'**un ou plusieurs produits auxiliaires sont choisis dans le groupe constitué de l'huile de jojoba, de l'huile d'olive, de l'huile de tournesol, de l'huile de soja, de l'huile d'arachide, de l'huile de colza, de l'huile d'amandes, de l'huile de palme, de l'huile de noix de coco, de l'huile de palmiste.

8. Composition dermatologique pour l'utilisation topique selon l'une des revendications 1 à 7, **caractérisée en ce qu'**un émulgateur huile/eau, notamment un hydrolysat de protéine végétale, est contenu.

9. Produit pharmaceutique contenant une composition dermatologique selon l'une des revendications 1 à 8 pour l'utilisation topique dans la prophylaxie et le traitement de maladies de la peau sélectionnées dans le groupe constitué de l'acné, de la séborrhée, de l'eczéma atopique (neurodermite), de l'hirsutisme, du psoriasis (psoriasis), ainsi que de la peau sèche /atopique.
